# EUROPÄISCHE PATENTSCHRIFT

(11) **EP 2 528 549 B1**
(45) Veröffentlichungstag und Bekanntmachung des Hinweises auf die Patenterteilung: **05.06.2013**
(21) Anmeldenummer: 11701742.6
(22) Anmeldetag: 06.01.2011
(51) Int. Cl.: A61F 2/58, A61F 2/68

(54) **VERSTELLEINRICHTUNG UND VERFAHREN ZUM BETREIBEN EINER VERSTELLEINRICHTUNG**
ADJUSTING MECHANISM AND METHOD OF OPERATING SUCH AN ADJUSTING MECHANISM
DISPOSITIF DE DÉPLACEMENT ET PROCÉDÉ POUR LE FONCTIONNEMENT D'UN DISPOSITIF DE DÉPLACEMENT

(30) Priorität: 25.01.2010 DE 102010005690
(43) Veröffentlichungstag der Anmeldung: 05.12.2012
(73) Patentinhaber: Otto Bock Healthcare Products GmbH, 1070 Wien (AT)
(72) Erfinder: INSCHLAG, Josef, A-8251 St. Lorenz am Wechsel (AT); EDER, Marcus, A-1220 Wien (AT)
(74) Vertreter: Stornebel, Kai
(86) Internationale Anmeldenummer: PCT/EP2011/000022
(87) Internationale Veröffentlichungsnummer: WO 2011/088964

(56) Entgegenhaltungen:
- WO-A1-96/41383
- WO-A1-2007/063266
- WO-A2-2006/076164
- BACHER J ET AL: "Actions to increase the Cogging Torque and Effects of the increased Cogging Torque to Permanent Magnet DC Motors", POWER ELECTRONICS AND APPLICATIONS, 2005 EUROPEAN CONFERENCE ON DRESDEN, GERMANY 11-14 SEPT. 2005, PISCATAWAY, NJ, USA,IEEE, PISCATAWAY, NJ, USA, 11. September 2005 (2005-09-11), Seiten P.1-P.7, XP010932656, DOI: DOI:10.1109/EPE.2005.219535 ISBN: 978-90-75815-09-2

## Beschreibung

Die Erfindung betrifft eine Verstelleinrichtung für eine prothetische Einrichtung mit einem Antrieb zur Verstellung zumindest einer ersten Komponente der prothetischen Einrichtung relativ zu einer zweiten Komponente, wobei der Antrieb als ein permanent erregter Elektromotor ausgebildet ist und einen Stator mit Erregerspulen und einen Rotor mit zumindest einem Permanentmagneten als Ankermagnet aufweist. Weiterhin betrifft die Anmeldung eine prothetische Einrichtung mit einer solchen Verstelleinrichtung sowie ein Verfahren zum Betreiben einer solchen Verstelleinrichtung.

Verstellantriebe werden in der Orthopädietechnik umfangreich eingesetzt, beispielsweise in angetriebenen Prothesen wie Handprothesen oder Ellenbogenprothesen. Dabei ist es vorgesehen, dass die Verstelleinrichtung an einer Komponente der Prothese angeordnet ist und eine zweite Komponente relativ zu der ersten Komponente verlagert. Auf diese Weise ist es möglich, beispielsweise einen Prothesenunterarm relativ zu einem Oberarmstumpf zu verlagern, ebenfalls können in einer Prothesenhand die Prothesenfinger bewegt werden, beispielsweise um eine Greifbewegung auszuführen.

Um den Antrieb nicht eingeschaltet lassen zu müssen, um die einmal erreichte Position zu erhalten, sind Einrichtungen zur mechanischen Blockierung der Komponenten zueinander vorgesehen. Solche Einrichtungen können Bremsen sein, die die Komponenten in der einmal erreichten Stellung verriegeln. Die Verriegelung oder Sperrung durch mechanische Komponenten hat den Nachteil hoher Herstellkosten, die sich vor allem durch die erhöhten Materialkosten und die vergrößerte Teilanzahl ergeben. Darüber hinaus verschleißen mechanische Komponenten, was zu erhöhten Wartungskosten führen kann, und sie benötigen relativ viel Bauraum.

Die DE 10 2005 061 313 A1 beschreibt eine Handprothese mit einem Chassis, an dem mehrere Fingerprothesen gelenkig belagert sind, die über einen Antrieb um zumindest eine Schwenkachse relativ zu dem Chassis bewegbar sind. Der Antrieb ist als ein Elektromotor ausgeführt.

Aufgabe der vorliegenden Erfindung ist es, eine Verstelleinrichtung für eine prothetische Einrichtung, eine prothetische Einrichtung als solche sowie ein Verfahren zum Betreiben eine Verstelleinrichtung bereitzustellen, mit denen die beschriebenen Nachteile vermieden werden.

Erfindungsgemäß wird diese Aufgabe durch eine Verstelleinrichtung, eine prothetische Einrichtung sowie ein Verfahren gemäß den unabhängigen Ansprüchen gelöst. Vorteilhafte Ausgestaltungen und Weiterbildungen der Erfindung sind in den Unteransprüchen aufgeführt.

Die erfindungsgemäße Verstelleinrichtung für eine prothetische Einrichtung mit einem Antrieb zur Verstellung zumindest einer ersten Komponente der prothetischen Einrichtung relativ zu einer zweiten Komponente, wobei der Antrieb als ein permanent erregter Elektromotor ausgebildet ist und einen Stator mit Erregerspulen und einen Rotor mit zumindest einem Permanentmagneten als Ankermagnet aufweist, sieht vor, dass an dem Stator zumindest ein Haltemagnet in Gestalt eines Permanentmagneten zur Ausbildung eines Rastmomentes für den Rotor angeordnet ist. Damit wird erreicht, dass auch nach Abschalten der Erregerspulen, beispielsweise nach dem Abschalten des Motors nach dem Schließen einer Prothesenhand, die einmal eingestellte Stellung der Komponenten zueinander erhalten bleibt. Der Haltemagnet wirkt mit dem Permanentmagneten des Rotors dergestalt zusammen, dass ein Rastmoment ausgebildet ist, so dass der Rotor in der eingenommenen Stellung, gegebenenfalls nach einem notwendigen Ausrichten des Rotors zu dem Haltemagneten, gehalten bleibt. Das Rastmoment des permanent erregten Elektromotors wird somit erhöht, so dass der Antrieb nach dem Abschalten der Erregerspannung blockiert wird. Dies geschieht ohne zusätzlichen Energieaufwand, ohne einen mechanischen Verschleiß und ohne einen vergrößerten Bauteilaufwand. Je größer die magnetischen Kräfte zwischen dem Haltemagnet und dem Ankermagnet sind, desto größer ist das Rastmoment, so dass ein Drehen des Motors durch Aufbringen externer Kräfte bis zu einem bestimmten Moment nicht mehr möglich ist. Die Höhe des Rastmomentes kann durch Auswahl der Magnete eingestellt werden.

Es kann vorgesehen sein, dass mehrere Haltemagnete zueinander beabstandet an dem Stator angeordnet sind, bevorzugt zwischen den Erregerspulen, um einerseits das Rastmoment zu erhöhen und andererseits einen unruhigen Motorlauf beim herkömmlichen Betrieb, also während des Verstellens, zu vermeiden. Um einen unruhigen Motorlauf zu vermeiden, können die Haltemagnete in Umfangsrichtung gleichmäßig zueinander verteilt angeordnet sein, ebenso können sie radial gleichmäßig von der Drehachse beabstandet angeordnet sein, also auf einem gemeinsamen Umfang liegen.

Ebenfalls können mehrere Ankermagnete mit über den Umfang abwechselnder Polarität in dem Rotor angeordnet sein, was einerseits den Gleichlauf erhöht und andererseits die Positioniergenauigkeit der Verstelleinrichtung erhöht, da dadurch die Wahrscheinlichkeit erhöht wird, dass ein Haltemagnet mit passender Polarität einem entsprechenden Ankermagneten gegenüber ausgerichtet ist.

Um das erhöhte Rastmoment durch den Haltemagneten oder die Haltemagneten beim normalen Betrieb des Motors zu kompensieren, wird die Wirkung des Magneten durch Überlagerung eines Gleichfeldes über das Drehfeld kompensiert. Dazu sind entsprechende Einrichtungen vorgesehen, die das Rastmoment während der Verstellung kompensieren können, beispielsweise ein Elektromagnet, der entsprechend angeordnet und ausgebildet ist. Dieser Elektromagnet kann auch der Stator selbst sein. So kann zur Kompensation des Magnetfeldes des Haltemagneten die Statorwicklung des Elektromotors eingesetzt werden. Das Gleichfeld zur Kompensation kann entweder durch zusätzliche Einrichtungen oder vorzugsweise durch Überlagerung eines Gleichstromes zum Motorstrom, also dem Drehfeld, erfolgen.

Die prothetische Einrichtung mit einer Verstelleinrichtung, wie sie oben beschrieben wurde, kann als Hand- oder Ellenbogenprothese ausgebildet sein, wobei die erste Komponente als ein Prothesenfinger und die zweite Komponente als ein Chassis ausgebildet sein kann. Ebenfalls ist es möglich, dass die erste Komponente als ein Unterarmschaft und die zweite Komponente als ein Oberarmschaft oder einer Aufnahmeeinrichtung für einen Oberarmstumpf ausgebildet ist.

Der Antrieb ist bevorzugt an einem Chassis gelagert und kann über ein Getriebe mit dem Prothesenfinger oder dem Unterarmschaft gekoppelt sein.

Das Verfahren zum Betreiben einer Verstelleinrichtung, wie sie oben beschrieben wurde, sieht vor, dass beim Anlegen eines Drehfeldes zum Antreiben des Rotors zumindest ein Gleichfeld dem Drehfeld überlagert wird, so dass das durch den Haltemagnet oder die Haltemagnete erzeugte Rastmoment kompensiert wird. Das Gleichfeld kann dabei beispielsweise über einen Elektromagneten ausgebildet werden.

Nachfolgend wird ein Ausführungsbeispiel der Erfindung anhand der beigefügten Figur näher erläutert. Es zeigen:
- Figur 1: einen Antrieb als Innenläufer in schematischer Darstellung;
- Figur 2: ein Anwendungsbeispiel in Gestalt einer Armprothese; sowie
- Figur 3: einen Antrieb als Außenläufer.

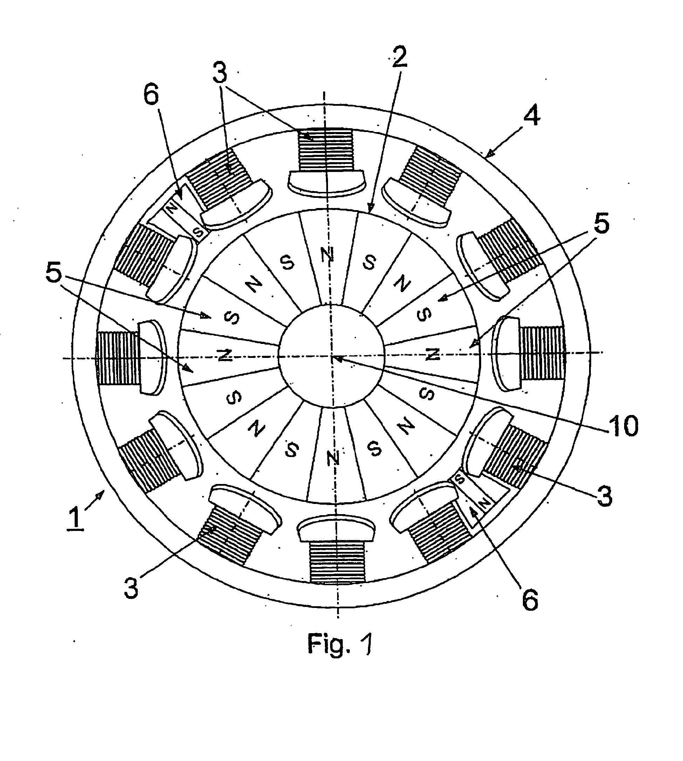

Die Figur 1 zeigt schematisch einen Antrieb 1 einer Verstellvorrichtung mit einem Stator 4, an dessen inneren Umfang Erregerspulen 3 angeordnet sind. Vorliegend sind zwölf Erregerspulen 3 vorgesehen, die gleichmäßig über den Umfang des Stators 4 angeordnet sind. Innerhalb des Stators 4 ist ein Rotor 2 angeordnet, der mehrere Permanentmagnete 5 unterschiedlicher Polarität aufweist. Die außen angeordneten Pole der Ankermagnete 5 weisen abwechselnde Polaritäten auf. Der Rotor 2 dreht sich bei Anlegen eines Drehfeldes durch die Erregerspulen 3 um eine Drehachse 10. Zwischen zwei Erregerspulen 3 sind im dargestellten Ausführungsbeispiel zwei Haltemagnete 6 in Gestalt von Permanentmagneten angeordnet, die ein Rastmoment erzeugen, ohne dass Energie zugeführt werden muss. Die Ankermagnete 5 richten sich dann entsprechend ihrer Polarität den Haltemagneten 6 gegenüber aus, wobei es zu geringfügigen Verdrehungen des Rotors 2 um die Drehachse 10 kommen kann. Nach Abschalten der Erregerspulen 3 wird über die Haltemagnete 6 der Rotor 2 in der jeweiligen Position gehalten, so dass beispielsweise nach dem Schließen einer Prothesenhand die Griffkraft auch nach dem Abschalten der Erregerspulen 3 ohne zusätzlichen Energieaufwand aufrecht erhalten bleibt.

Im dargestellten Ausführungsbeispiel sind zwei Haltemagnete 6 einander gegenüberliegend angeordnet. Grundsätzlich können auch weitere Haltemagnete 6 zwischen den Erregerspulen 3 angeordnet sein. Ebenfalls ist es möglich, nur mit einem Haltemagnet 6 ein ausreichendes Rastmoment bereitzustellen, sofern die magnetische Feldstärke ausreicht.

Um das durch die Haltemagnete 6 erhöhte Rastmoment beim normalen Betrieb des Antriebs 1 zu kompensieren, kann ein Gleichfeld durch nicht dargestellte Elektromagnete dem Magnetfeld der Haltemagnete 6 überlagert werden, so dass keine periodisch auftretenden Schwankungen im Rundlauf des Motors auftreten.

Die durch die Haltemagnete 6 bewirkte elektromagnetische Sperre kann mit sehr niedrigen Kosten realisiert werden und ist praktisch verschleißfrei, wodurch die Wartungskosten gering gehalten werden.

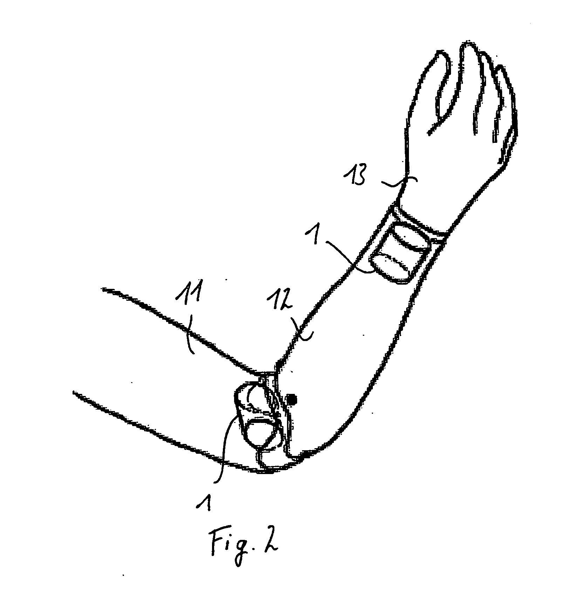

In der Figur 2 ist schematisch eine Armprothese mit einem Oberarmschaft 11 dargestellt, der an einem Oberarmstumpf festlegbar ist. An dem Oberarmschaft 11 ist ein Unterarmschaft 12 gelenkig befestigt, an dessen distalem Ende wiederum eine Prothesenhand 13 angeordnet ist. Zwischen dem Oberarmschaft 11 und dem Unterarmschaft 12 ist ein Antrieb 1, wie er oben beschrieben ist, angeordnet. Über einen solchen Antrieb 1 ist es möglich, den Unterarmschaft 12 relativ zu dem Oberarmschaft 11 gesteuert zu verschwenken. Die Verschwenkung kann beispielsweise auf der Grundlage myoelektrischer Signale oder einer anderen Steuereinrichtung erfolgen. Steht der Unterarmschaft 12 relativ zu dem Oberarmschaft 11 in der gewünschten Stellung, wird der Antrieb 1 abgeschaltet. Über die in dem Antrieb 1 angeordneten Haltemagnete 6 ist es möglich, ein erhöhtes Rastmoment des Antriebs 1 bereitzustellen, so dass der Unterarmschaft 12 in der Position relativ zu dem Oberarmschaft 11 gehalten werden kann. Zumindest ist es möglich, das Rastmoment soweit zu erhöhen, dass sich der Unterarmschaft 12 zusammen mit der Prothesenhand 13 nicht ohne weitere äußere Kräfte relativ zu dem Oberarmschaft 11 bewegt, es kann so eine Haltefunktion entgegen der Schwerkraft und den auftretenden Trägheitskräften bei den üblichen Bewegungen bereitgestellt werden.

Eine eventuell erforderliche, zusätzliche formschlüssige Verriegelung kann in dem Gelenk zwischen dem Oberarmschaft 11 und dem Unterarmschaft 12 vorgesehen sein.

Ebenfalls kann in dem Gelenk zwischen dem Unterarmschaft 12 und der Prothesenhand 13 ein Antrieb 1 angeordnet sein, der Haltemagnete 6 aufweist, so dass eine Rotationsbewegung der Prothesenhand 13 relativ zu dem Unterarmschaft 12 erfolgen kann, wenn der Antrieb 1 aktiviert wird und eine Verrastung stattfindet, also eine Erhöhung des Rastmomentes, wenn der Antrieb 1 ausgeschaltet ist. Auch hier können ein oder mehrere zusätzliche Verriegelungseinrichtungen vorgesehen sein, um die Position der Prothesenhand 13 relativ zu dem Unterarmschaft 12 zu fixieren.

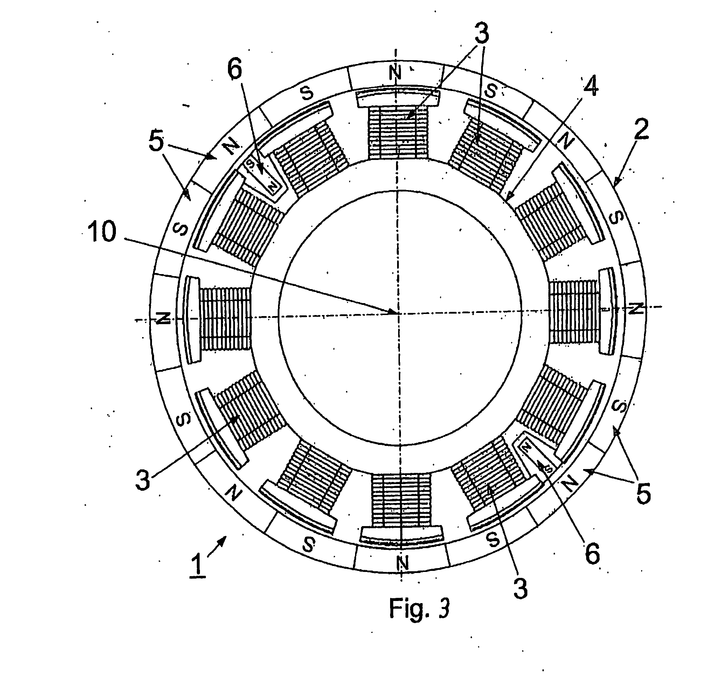

Eine Variante der Erfindung ist in der Figur 3 dargestellt, in der der Antrieb 1 als Außenläufermotor ausgebildet ist. Die Permanentmagnete 5 mit abwechselnd orientierter Polarität sind ringförmig um die still stehenden Erregerspule 3 angeordnet. Die Haltemagnete 6 sind wieder zwischen den Erregerspulen 3 angeordnet und bewirken das zusätzliche Rastmoment für den Rotor 2. Die Permanentmagnete 5 können in einer Glocke oder in einem Träger angeordnet sein, über die oder über den das Drehmoment abgeleitet und beispielsweise an ein Getriebe abgegeben wird.

Weiterhin besteht die Möglichkeit, dass der Antrieb sowohl gemäß Figur 1 als auch gemäß Figur 3 umgekehrt zu betreiben, also dass die Permanentmagneten 5 festgelegt sind und sich auf dem Stator 4 befinden und dass sich die Erregerspulen 3 zusammen mit den Haltemagneten 6 drehen und auf dem Rotor 2 angeordnet sind. Dadurch würde die Variante gemäß Figur 1 zum Außenläufer und die Variante gemäß Figur 3 zum Innenläufer.

## Patentansprüche

1. Verstelleinrichtung für eine prothetische Einrichtung mit einem Antrieb (1) zur Verstellung zumindest einer ersten Komponente der prothetischen Einrichtung relativ zu einer zweiten Komponente, wobei der Antrieb (1) als ein permanenterregter Elektromotor ausgebildet ist und einen Stator (4) mit Erregerspulen (3) und einen Rotor (2) mit zumindest einem Permanentmagneten als Ankermagnet (5) aufweist, **dadurch gekennzeichnet, dass** an dem Stator (4) zumindest ein Haltemagnet (6) in Gestalt eines Permanentmagneten zur Ausbildung eines Rastmomentes für den Rotor (2) angeordnet ist.

2. Verstelleinrichtung nach Anspruch 1, **dadurch gekennzeichnet, dass** mehrere Haltemagnete (6) zueinander beabstandet an dem Stator (4) angeordnet sind.

3. Verstelleinrichtung nach Anspruch 1 oder 2, **dadurch gekennzeichnet, dass** mehrere Ankermagnete (5) mit über den Umfang abwechselnder Polarität in dem Rotor (2) angeordnet sind.

4. Verstelleinrichtung nach einem der voranstehenden Ansprüche, **dadurch gekennzeichnet, dass** Einrichtungen zur Überlagerung des Drehfeldes mit einem magnetischen Gleichfeld zur Kompensation des Rastmomentes während der Verstellung vorgesehen sind.

5. Prothetische Einrichtung mit einer Verstelleinrichtung nach einem der voranstehenden Ansprüche.

6. Prothetische Einrichtung nach Anspruch 5, **dadurch gekennzeichnet, dass** sie als Hand- oder Ellenbogenprothese ausgebildet ist.

7. Prothetische Einrichtung nach Anspruch 5 oder 6, **dadurch gekennzeichnet, dass** die erste Komponente ein Prothesenfinger und die zweite Komponente ein Chassis ist.

8. Prothetische Einrichtung nach Anspruch 7, **dadurch gekennzeichnet, dass** der Antrieb an dem Chassis gelagert und über ein Getriebe mit dem Prothesenfinger gekoppelt ist.

9. Verfahren zum Betreiben einer Verstelleinrichtung nach einem der voranstehenden Ansprüche, **dadurch gekennzeichnet, dass** bei Anlegen eines Drehfeldes zum Antreiben des Rotors (2) zumindest ein Gleichfeld dem Drehfeld überlagert so, so dass das durch den Haltemagnet (6) oder die Haltemagnete (6) erzeugte Rastmoment kompensiert wird.

## Claims

1. An adjusting device for a prosthetic device, with a drive (1) for adjusting at least one first component of the prosthetic device relative to a second component, wherein the drive (1) is designed as a permanent-magnet electric motor and has a stator (4) with exciter coils (3) and a rotor (2) with at least one permanent magnet as armature magnet (5), **characterized in that** at least one holding magnet (6) in the form of a permanent magnet is arranged on the stator (4) in order to provide a cogging torque for the rotor (2).

2. The adjusting device as claimed in claim 1, **characterized in that** a plurality of holding magnets (6) are arranged, spaced apart from one another, on the stator (4).

3. The adjusting device as claimed in claim 1 or 2, **characterized in that** a plurality of armature magnets (5) are arranged in the rotor (2), with alternating polarity about the circumference.

4. The adjusting device as claimed in one of the preceding claims, **characterized in that** devices are provided for superposing the rotary field with a constant magnetic field for compensation of the cogging torque during the adjustment.

5. A prosthetic device with an adjusting device as claimed in one of the preceding claims.

6. The prosthetic device as claimed in claim 5, **characterized in that** it is designed as a prosthetic hand or prosthetic elbow,

7. The prosthetic device as claimed in claim 5 or 6, **characterized in that** the first component is a prosthetic finger and the second component is a chassis.

8. The prosthetic device as claimed in claim 7, **characterized in that** the drive is mounted on the chassis and is coupled to the prosthetic finger via a gear.

9. A method for operating an adjusting device as claimed in one of the preceding claims, **characterized in that**, when a rotary field is applied to drive the rotor (2), at least one constant field is superposed on the rotary field, such that the cogging torque generated by the holding magnet (6) or the holding magnets (6) is compensated.

## Revendications

1. Dispositif de déplacement pour un dispositif prothétique, comprenant un entraînement (1) pour déplacer au moins un premier composant du dispositif prothétique relativement à un deuxième composant, l'entraînement étant réalisé sous la forme d'un moteur électrique excité de façon permanente et présentant un stator (4) avec bobines d'excitation (3) et un rotor (2) comportant au moins un aimant permanent en tant qu'aiment d'induit (5), **caractérisé en ce que** sur le stator (4) est agencé au moins un aimant de maintien (6) sous la forme d'un aimant permanent pour générer un moment de maintien pour le rotor (2).

2. Dispositif de déplacement selon la revendication 1, **caractérisé en ce que** plusieurs aiments de maintien (6) espacés les uns des autres sont agencés sur le stator (4).

3. Dispositif de déplacement selon la revendication 1 ou 2, **caractérisé en ce que** plusieurs aimants d'induit (5) sont disposés dans le rotor (2) avec des polarités alternées sur le pourtour.

4. Dispositif de déplacement selon l'une des revendications précédentes, **caractérisé en ce que** des moyens sont prévus pour superposer au champ tournant un champ magnétique continu pour compenser le moment de maintien pendant le déplacement.

5. Dispositif prothétique comprenant un dispositif de déplacement selon l'une des revendications précédentes.

6. Dispositif prothétique selon la revendication 5, **caractérisé en ce qu'**il est réalisé en tant que prothèse de la main ou du coude.

7. Dispositif prothétique selon la revendication 5 ou 6, **caractérisé en ce que** le premier composant est un doigt prothétique et le second composant un châssis.

8. Dispositif prothétique selon la revendication 7, **caractérisé en ce que** l'entraînement est supporté sur le châssis et couplé ou doigt prothétique par l'intermédiaire d'une transmission.

9. Procédé pour faire fonctionner un dispositif de déplacement selon l'une des revendications précédentes, **caractérisé en ce que** pendant l'application d'un champ tournant pour entraîner le rotor (2) au moins un champ continu est superposé au champ tournant de sorte que le moment de maintien produit par l'aimant de maintien (6) ou les aimants de maintien (6) est compensé.
